# EUROPEAN PATENT SPECIFICATION

(11) **EP 4 155 416 B1**
(45) Date of publication and mention of the grant of the patent: **10.01.2024**
(21) Application number: 21198504.9
(22) Date of filing: 23.09.2021
(51) Int. Cl.: C12Q 1/6806, C12Q 1/6834

(54) **METHOD FOR OBTAINING SPATIAL AND SEQUENCING INFORMATION OF M-RNA FROM TISSUE**
VERFAHREN ZUR ERLANGUNG VON RAUM- UND SEQUENZIERUNGSINFORMATIONEN VON M-RNA AUS GEWEBE
PROCÉDÉ D'OBTENTION D'INFORMATIONS SPATIALES ET DE SÉQUENÇAGE D'ARN-M À PARTIR DE TISSU

(43) Date of publication of application: 29.03.2023
(73) Proprietor: Miltenyi Biotec B.V. & Co. KG, 51429 Bergisch Gladbach (DE)
(72) Inventor: PINARD, Robert, 51429 Bergisch Gladbach (DE); MEYER, Hansueli, 51429 Bergisch Gladbach (DE); ROTHMANN, Thomas, 51429 Bergisch Gladbach (DE); NEHME, Chris, 51429 Bergisch Gladbach (DE)
(74) Representative: Kisters, Michael Marcus

(56) References cited:
- US-A1- 2020 399 687
- US-A1- 2021 155 982

## Description

### BACKGROUND

The present invention is directed to a process for sequencing and spatial decoding of m-RNA molecules of a tissue.

It has been challenging to detect all expressed genes on a sub cellular level on tissue by keeping the spatial information and afterwards sequences those genes for potential variants. It is especially challenging to reach sub cellular resolution.

For example, the development of a sequencing system for spatial decoding of DNA barcode molecules is described by "Single-molecule resolution" (Nature, Dec 2020, Yusuke Oguchi)

A commercial approach is available wherein the spatial information is maintained as the tissue RNA molecules are tagged with a spatial identifier pre-spotted on an array. The resulting libraries are later sequenced by standard in vitro NGS (next generation sequencing) sequencing techniques. With the spotting process the position of the spatial identifier on the array is known before the sequencing process takes place. After sequencing of the spatial identifier, the linked RNA sequence of interest can be assigned to the tissue location. One major limitation of this approach is the resolution of the technology as it is dependent on the feature size of the spots on the array which is currently on a multicellular level only.

Technologies to obtain sequencing and spatial information are for example published in US20150344942A1, WO2016162309A1 and WO2012/140224.

Single-cell transcriptome analysis has been revolutionized by DNA barcodes that index cDNA libraries, allowing highly multiplexed analyses to be performed. Furthermore, DNA barcodes are being leveraged for spatial transcriptomes. Although spatial resolution relies on methods used to decode DNA barcodes, achieving single-molecule (m-RNA) decoding remains a challenge.

### SUMMARY

It was therefore an object of the invention to provide a method to provide a method for sequencing and spatial decoding of m-RNA molecules of a tissue where the generation and decoding of the DNA barcode are done concomitantly.

Object of the invention is therefore a method to obtain the spatial location and sequence information of an m-RNA target sequence on a tissue sample comprising the steps
a. providing a solid surface having at least with at least one fiducial marker
b. attaching a plurality of anchor molecules comprising a photo cleavable linker and an adapter unit to the solid surface
c. Binding scaffolding molecules comprising a unit capable of binding to the adapter unit of an anchor molecule, a poly-inosine unit having 5 to 30 inosine bases and a poly-adenine unit having 10 to 50 adenine bases to the anchor molecules
d. randomly incorporating adenine, guanine, cytosine and thymine as nucleic bases to the anchor molecule complementing the inosine bases of the scaffolding molecules optionally during sequencing-by-synthesis (SBS) thereby creating barcodes on the anchor molecules, wherein the nucleic bases are provided with a photo-detectable unit and wherein the sequence of the barcodes (and optionally their generation) and their spatial location relative to the fiducial marker is detected simultaneously as spatial information.
e. incorporating thymine to the anchor molecules complementing the poly-adenine unit of the scaffolding molecules thereby creating a poly-T unit
f. removing the scaffolding molecules from the anchor molecules
g. A tissue sample that has been provided comprises at least one m-RNA strand wherein at least one m-RNA strand of the sample binds to a poly-T unit of at least one anchor molecule
h. reverse transcription of the m-RNA strand creating a c-DNA strand attached to the solid surface
i. remove c-DNA strands from the solid surface by cleaving the photo-cleavable linker of the anchor molecules
j. obtaining the sequence information of the c-DNA strands and linking the spatial information with the sequence information of the c-DNA strands.

### BRIEF DESCRIPTION OF THE DRAWINGS

Fig. 1 shows a generic workflow of the invention
Fig. 2 shows the build-up of the barcode section at the anchor molecules

### DETAILED DESCRIPTION

The here described method is being used to detect mRNA on tissue with a very high spatial resolution of 50 to 300 nm.

The method of the invention is described in the following in more detail according the process steps which have to be performed in the sequence a) to j).

### Step a) providing a solid surface having at least with at least one fiducial marker

The solid substrate is preferable provided with a functionalized surface. The surface may be functionalized with commonly used chemistries available for solid support attachment of oligonucleotides containing a photo cleavable linker and optionally assembled together with other parts to form a microfluidic flow cell with an inlet and outlet.

The functionalized surface may include for example amine-modified oligos covalently linked to an activated carboxylate group or succinimidyl ester, thiol-modified oligos covalently linked via an alkylating reagent such as an iodoacetamide or maleimide, acryditeTM modified oligos covalently linked through a thioether , digoxigenin NHS Ester or biotin-modified oligos captured by immobilized Streptavidin which is able to interact with the biotin.

Optionally, the anchor molecules are randomly distributed on the solid substrate with a density corresponding to the molecule concentration.

The anchor molecule may further comprise at least one primer sequence for the polymerase capable of rolling circle amplification.

### Step b) attaching a plurality of anchor molecules a photo cleavable linker to the functionalized solid surface

Afterwards the pre-manufactured anchor molecules are loaded on the solid surface with a buffer solution. The anchor molecules will interact with the functionalized surface and randomly spatially distribute on the entire area.

Preferable, anchor molecules are generated having a photo cleavable linker, an adapter unit like a P5 or P7 adapter or other short sequences. Billions of such single molecules can be manufactured. The density of the anchor molecules can be controlled by the concentration loaded.

### Step c) Binding scaffolding molecules comprising a unit capable of binding to the adapter unit of an anchor molecule, a poly-inosine unit having 5 to 30 inosine bases and a poly-adenine unit having 10 to 50 adenine bases to the anchor molecules

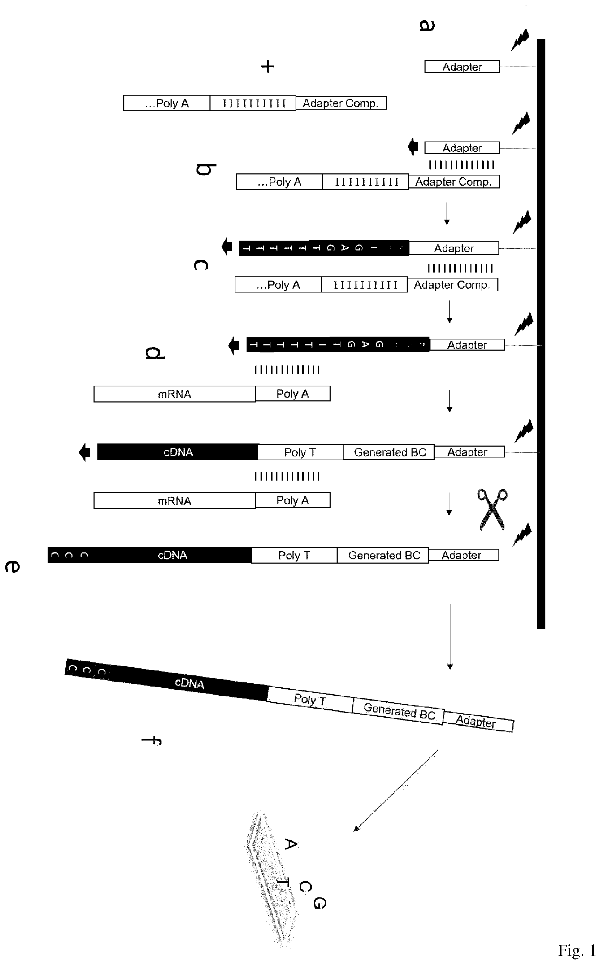

The scaffolding molecules are shown in Fig. 1 a and b and bind via the adapter unit to the anchor molecule. Suitable adapters and their counterparts are known to the person skilled in the art, like P5 or P7.

The poly-inosine unit serves as the template for the generation of randomized barcode and may preferable have 5 to 30 inosine bases

Step d) randomly incorporating adenine, guanine, cytosine and thymine as nucleic bases to the anchor molecule complementing the inosine bases of the scaffolding molecules optionally during sequencing-by-synthesis (SBS) thereby creating barcodes on the anchor molecules, wherein the nucleic bases are provided with a photo-detectable unit and wherein the sequence of the barcodes (and optionally their generation) and their spatial location relative to the fiducial marker is detected simultaneously as spatial information.

Preferable, randomly incorporating adenine, guanine, cytosine and thymine as nucleic bases to the anchor molecule is performed by providing a mixture of A, T, G and C and a polymerase.

During this step, random barcodes are created on the anchor molecules which are used to simultaneously identifying and ascribing their spatial location relative to the fiducial markers

In this step, the inosine bases of the scaffolding molecule are complemented with adenine, guanine, cytosine and thymine (A, C, G, T) in random fashion to create a barcode sequence. To this end, the nucleic bases are provided with a photo-detectable unit. Such nucleic bases are known from the sequence-by-synthesis and are commercial available. The in random fashion is established by providing a mixture of A, T, G and C and a polymerase to the sample.

Due to the photo-detectable units, the generation of the barcode can easily be monitored by taking appropriate images.

The spatial information is preferable obtained by detecting the photo-detectable unit of the nucleic bases and/or by sequencing-by-synthesis.

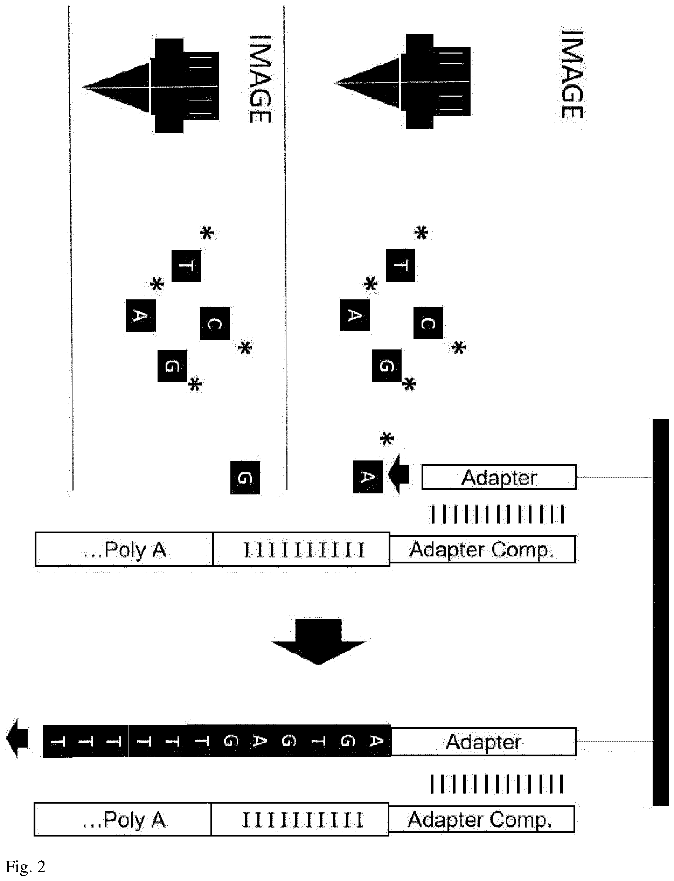

As shown in Fig. 2, an image is taken after each extension cycle to determine the incorporated base. The image may have super resolution on order to distinguish each barcode.

The bases A, C, G, T will randomly incorporate on each individual single molecule in each cycle and therefore creating a unique spatial single molecular identifier. The solid substrate also contains at least 2 independent fiducial marks that could be fluorescent or auto fluorescent markers. Images are also taken of those fiducial marks and the X-Y position of the incorporated base is determined relative to the markers. In this manner, each sequence of each individual single molecule is recorded and the incorporated base its spatial location relative to the fiducial marks is stored as special information (X-Y distances).

Step e) incorporating thymine to the anchor molecules complementing the poly-adenine unit of the scaffolding molecules thereby creating a poly-T unit

After sequencing, the bases of the poly-A tails are filled with unlabelled T nucleotides. This step allows forming a double stranded DNA molecule which is stable.

Of course, the mixture of bases form the preceding steps has to be removed and optionally after washing, thymine and polymerase is provided

### Step f) removing the scaffolding molecules from the anchor molecules

As a next step, the solid substrate with the double stranded DNA complex is heat denatured to remove the scaffolding molecule forming a single strand oligonucleotide on the solid surface.

Step g) A tissue sample that has been provided comprises at least one m-RNA strand wherein at least one m-RNA strand of the sample binds to a poly-T unit of at least one anchor molecule
A tissue sample is brought in contact with the solid substrate where all the single molecules are located and permeabilized to release the mRNA molecules. The 30-50 bases poly T tail of the oligonucleotides, located in the vicinity will then hybridized to the expressed mRNA in the tissue via standard DNA/mRNA interaction.

Optionally, the tissue sample is permeabilized after providing to the surface.

### Step h) reverse transcription of the m-RNA strand creating a c-DNA strand attached to the solid surface

In the next steps, all the anchor molecules with the mRNA are reversely transcribed into cDNA and subsequently the tissue can be removed from the solid substrate using enzymatic or via chemical reactions.

The c-DNA stand may be circularized and then multiplied by a polymerase capable of rolling circle amplification into a plurality of DNA concatemers.

Optionally, only a subset of the mRNA (region of interest or ROI) can be reverse transcribed if only a predefine subset of the anchors are kept by removing unwanted areas using targeted light prior to the cDNA generation.

Regions of interest (ROI) on the tissue can be define using with an external laser UV light source using a digital micromirror device (DMD). Each ROI will get a certain dose of UV laser light and all the photo cleavable bonds of the single molecules attached to the solid substrate will be cut and removed.

### Step j) Remove c-DNA strands from the solid surface by cleaving all the photo-cleavable linker of the anchor molecules

Optionally, only a subset of the cDNA can be removed using targeted external laser UV light source to a region of interest on the solid surface as described above.

Step i) Obtaining the sequence information of the c-DNA strands and linking the spatial barcode sequence information of the c-DNA strands to the solid surface barcode.

Further processes may be done to prepare the cDNA for a sequencing library. In the last step each single molecule with the unique spatial anchor molecular identifier is sequenced in vitro and linked to the original determined location on the tissue. As the sequence of the barcodes and their spatial location relative to the fiducial marker is known from step d), the spatial information of the m-RNA can be easily determined. Since the sequence information of the c-DNA strands includes the sequence of the barcodes and the sequence information of the generated barcode on the solid surface is known from step d) and i) the two barcodes sequences can be matched to identify the origin/ spatial information of the mRNA on the tissue.

The approach of the invention is best suited for sequencing the 3'end of mRNAs captured.

Many applications for mRNA sequencing also require to identify the 5'end of the mRNA. The following workflow (also applicable for solid support) is solving this problem as visualized in the Figure 2 . B revers to universal bases like Inosine.

Optionally, the tissue may be further characterized by identification of different proteins expressed on the tissue, for example with antibody-conjugated dyes, preferable with the MACSima technology.

The method of the invention is in theory capable of producing 30 types of DNA barcode molecules with an average read length of ~20 nt with an error rate of less than 5% per nucleotide. This is sufficient to spatially identify them. Additionally, spatially identified DNA barcode molecules bound to antibodies can be detected at single-molecule resolution.

### Example of workflow:

Load identical oligonucleotides as anchor molecules consisting of a photo cleavable linker with a P5 adapter with 20-30 bases of Inosine and 30-50 bases of poly A base tail on to a functionalized solid surface (e.g. standard cover glass 25x75xlmm)
The anchor molecules will immobilize randomly on functionalized surface. Density of anchor molecules on surface controlled via concentration
The anchor molecules can have a minimal distance from each other of about 50-300 nm
25-30 bases of Inosine will theoretically allow 1.2×10^15 to 1.5×10^18 possible combinations on randomly incorporating labeled single nucleotides consisting of A, C, T and G when doing sequencing by synthesis (SBS)

Do sequencing by synthesis for 25-30 cycles and determine each base which was incorporated on each single molecule on the Inosine complementary base. Fig. 1 is showing the sequencing cycle. It shows the extension step where the first round of labeled nucleotides are incorporated to the Inosine base.

Sequencing by synthesis can be done with a dedicated super resolution optical system. The location information for each single molecule on the tissue slice holder will be written into a data base and can be downloaded by the user (website, cloud) or shipped with a consumable.

After sequencing all bases of the Inosine each single molecule will have a spatial unique molecular identifier.

Each location of the single molecule with the spatial unique molecular identifier can be determined to a relative position on the functionalized solid surface (e.g. fiducial mark)
The solid substrate also contains 2 independent fiducial marks. Images of those fiducial marks are taken and X-Y position are determined. Each sequence of each individual single molecule is recorded and the spatial location relative to the fiducial marks is stored as X-Y coordinates.

After sequencing the 25-30 bases, the poly A tails (~30 bases) are filled with unlabeled T nucleotides. This step will form a double stranded DNA molecule which is stable:
As a next step the solid substrate with double stranded molecules is denatured so that the double stranded DNA molecule is forming a single stranded oligonucleotide covalently attached to the surface.

A tissue sample is brought in contact with the solid substrate containing the single molecules and permeabilized . The expressed mRNA in the tissue will be released form the tissue following the permeabilization and their poly A tails will interact with the 30-50 bases poly T tail. In the next step, the tissue will be removed from the solid substrate.

The mRNA will be reverse transcribed using the anchored single molecule on the surface as the primer.

The cDNA containing uniquely generated, sequenced and pre-decoded, barcodes linked to the solid surface via a photo-cleavable linker will be released using targeted external laser UV light source will be further processed off the solid surface.

Further processes are done to prepare the cDNA for a sequencing library using commonly known approaches as end repair. A-tailing and adapter ligation.

In the last step each cDNA with the unique spatial single molecular identifier is sequenced in vitro and linked to the original determined location on the tissue.

Optionally the one could also further characterize the tissue sample and identify a few different proteins expressed on the tissue, for example with MACSima technology.

In addition, define regions of interest (ROI) on the tissue can be defined by removing unwanted portion available to mRNA hybridization using external UV laser light source using a digital micromirror device (DMD) to remove all the photo cleavable bonds of the single molecules attached to the solid substrate not wanted. Inversely, the cDNA of interested can also be released only from pre-determined ROI via the same principles but following the cDNA generation.

## Claims

1. A method to obtain the spatial location and sequence information of an m-RNA target sequence on a tissue sample comprising the steps
a. providing a solid surface having at least with at least one fiducial marker
b. attaching a plurality of anchor molecules comprising a photo cleavable linker and an adapter unit to the solid surface
c. Binding scaffolding molecules comprising a unit capable of binding to the adapter unit of an anchor molecule, a poly-inosine unit having 5 to 30 inosine bases and a poly-adenine unit having 10 to 50 adenine bases to the anchor molecules
d. randomly incorporating adenine, guanine, cytosine and thymine as nucleic bases to the anchor molecule complementing the inosine bases of the scaffolding molecules thereby creating barcodes on the anchor molecules, wherein the nucleic bases are provided with a photo-detectable unit and wherein the sequence of the barcodes and their spatial location relative to the fiducial marker is detected simultaneously as spatial information.
e. incorporating thymine to the anchor molecules complementing the poly-adenine unit of the scaffolding molecules thereby creating a poly-T unit
f. removing the scaffolding molecules from the anchor molecules
g. A tissue sample that has been provided comprises at least one m-RNA strand wherein at least one m-RNA strand of the sample binds to a poly-T unit of at least one anchor molecule
h. reverse transcription of the m-RNA strand creating a c-DNA strand attached to the solid surface
i. remove c-DNA strands from the solid surface by cleaving the photo-cleavable linker of the anchor molecules
j. obtaining the sequence information of the c-DNA strands and linking the spatial information with the sequence information of the c-DNA strands.

2. Method according to claim 1 **characterized in that** randomly incorporating adenine, guanine, cytosine and thymine as nucleic bases to the anchor molecule is performed by providing a mixture of A, T, G and C and a polymerase.

3. Method according to claim 1 or 2 **characterized in that** the spatial information is obtained by detecting the photo-detectable unit of the nucleic bases.

4. Method according to any of the claims 1 to 3 **characterized in that** the spatial information is obtained by sequencing-by-synthesis.

5. Method according to any of the claims 1 to 4 **characterized in that** the sample tissue is removed from the surface.

6. Method according to any of the claims 1 to 5 **characterized in that** the c-DNA stand is circularized and then multiplied by a polymerase capable of rolling circle amplification into a plurality of DNA concatemers.

7. Method according to any of the claims 1 to 6 **characterized in that** the anchor molecules are randomly distributed on the solid substrate with a density corresponding to the molecule concentration.

8. Method according to any of the claims 1 to 6 **characterized in that** the tissue sample is permeabilized after providing to the surface.

9. Method according to any of the claims 1 to 8 **characterized in that** the anchor molecule comprises at least one primer sequence for the polymerase capable of rolling circle amplification.

## Patentansprüche

1. Verfahren zur Erlangung der räumlichen Lage und Sequenzinformation einer m-RNA-Zielsequenz auf einer Gewebeprobe, umfassend die Schritte
a. Bereitstellen einer festen Oberfläche mit mindestens einem Referenzmarker
b. Anbringen einer Vielzahl von Ankermolekülen, die einen fotospaltbaren Linker und eine Adaptereinheit umfassen, an der festen Oberfläche
c. Binden von Gerüstmolekülen, umfassend eine Einheit, die fähig ist, an die Adaptereinheit eines Ankermoleküls zu binden, eine Polyinosineinheit mit 5 bis 30 Inosinbasen und eine Polyadenineinheit mit 10 bis 50 Adeninbasen, an die Ankermoleküle
d. zufälliges Einbauen von Adenin, Guanin, Cytosin und Thymin als Nukleinbasen in das Ankermolekül, die die Inosinbasen der Gerüstmoleküle komplementieren, wodurch Strichcodes auf den Ankermolekülen erzeugt werden, wobei die Nukleinbasen mit einer fotodetektierbaren Einheit bereitgestellt sind und wobei die Sequenz der Strichcodes und ihre räumliche Lage in Bezug auf den Referenzmarker gleichzeitig als räumliche Information detektiert wird
e. Einbauen von Thymin in die Ankermoleküle, das die Polyadenineinheit der Gerüstmoleküle komplementiert, wodurch eine Poly-T-Einheit entsteht
f. Entfernen der Gerüstmoleküle von den Ankermolekülen
g. eine Gewebeprobe, die bereitgestellt wurde, umfasst mindestens einen m-RNA-Strang, wobei mindestens ein m-RNA-Strang der Probe an eine Poly-T-Einheit von mindestens einem Ankermolekül bindet
h. Reverses Transkribieren des m-RNA-Strangs unter Bildung eines c-DNA-Strangs, der an die feste Oberfläche gebunden ist
i. Entfernen der c-DNA-Stränge von der festen Oberfläche durch Spaltung des fotospaltbaren Linkers der Ankermoleküle
j. Erlangen der Sequenzinformationen der c-DNA-Stränge und Verknüpfung der räumlichen Information mit den Sequenzinformation der c-DNA-Stränge.

2. Verfahren nach Anspruch 1, **dadurch gekennzeichnet, dass** das zufällige Einbauen von Adenin, Guanin, Cytosin und Thymin als Nukleinbasen in das Ankermolekül durch Bereitstellung einer Mischung aus A, T, G und C und einer Polymerase erfolgt.

3. Verfahren nach Anspruch 1 oder 2, **dadurch gekennzeichnet, dass** die räumliche Information durch Detektion der fotodetektierbaren Einheit der Nukleinbasen erlangt wird.

4. Verfahren nach einem der Ansprüche 1 bis 3,
**dadurch gekennzeichnet, dass** die räumliche Information mittels Sequenzierung durch Synthese erlangt wird.

5. Verfahren nach einem der Ansprüche 1 bis 4,
**dadurch gekennzeichnet, dass** das Probengewebe von der Oberfläche entfernt wird.

6. Verfahren nach einem der Ansprüche 1 bis 5,
**dadurch gekennzeichnet, dass** der c-DNA-Strang zirkularisiert und dann durch eine Polymerase, die zur Rolling-Circle-Amplifikation fähig ist, in eine Vielzahl von DNA-Konkatemeren vervielfältigt wird.

7. Verfahren nach einem der Ansprüche 1 bis 6,
**dadurch gekennzeichnet, dass** die Ankermoleküle zufällig auf dem festen Substrat mit einer Dichte verteilt werden, die der Molekülkonzentration entspricht.

8. Verfahren nach einem der Ansprüche 1 bis 6,
**dadurch gekennzeichnet, dass die** Gewebeprobe nach der Bereitstellung an die Oberfläche permeabilisiert wird.

9. Verfahren nach einem der Ansprüche 1 bis 8,
**dadurch gekennzeichnet, dass** das Ankermolekül mindestens eine Primersequenz für die Polymerase enthält, die zur Rolling-Circle-Amplifikation fähig ist.

## Revendications

1. Procédé pour obtenir la localisation spatiale et les informations de séquence d'une séquence cible d'ARNm sur un échantillon de tissu comprenant les étapes consistant à
a. fournir une surface solide ayant au moins avec au moins un marqueur fiduciaire
b. attacher une pluralité de molécules d'ancre comprenant un lieur photo clivable et une unité d'adaptateur à la surface solide
c. lier les molécules d'échafaudage comprenant une unité capable de se lier à l'unité d'adaptateur d'une molécule d'ancre, une unité poly-inosine ayant 5 à 30 bases inosine et une unité poly-adénine ayant 10 à 50 bases adénine aux molécules d'ancre
d. incorporer au hasard l'adénine, la guanine, la cytosine et la thymine comme bases nucléiques à la molécule d'ancre complémentant les bases inosine des molécules d'échafaudage créant ainsi des codes-barre sur les molécules d'ancre, les bases nucléiques étant fournies avec une unité photo-détectable et la séquence des codes-barre et leur localisation spatiale par rapport au marqueur fiduciaire étant détectées simultanément comme informations spatiales
e. incorporer la thymine aux molécules d'ancre complémentant l'unité poly-adénine des molécules d'échafaudage créant ainsi une unité poly-T
f. enlever les molécules d'échafaudage des molécules d'ancre
g. un échantillon de tissu qui a été fourni comprend au moins un brin d'ARNm au moins un brin d'ARNm de l'échantillon se liant à une unité poly-T d'au moins une molécule d'ancre
h. la transcription inverse du brin d'ARNm créant un brin d'ADNc attaché à la surface solide
i. enlever les brins d'ADNc de la surface solide en clivant le lieur photo-clivable des molécules d'ancre
j. obtenir les informations de séquence des brins d'ADNc et lier les informations spatiales avec les informations de séquence des brins d'ADNc.

2. Procédé selon la revendication 1, **caractérisé en ce que** l'incorporation aléatoire d'adénine, de guanine, de cytosine et de thymine comme bases nucléiques à la molécule d'ancre est effectuée en fournissant un mélange de A, T, G et C et une polymérase.

3. Procédé selon la revendication 1 ou 2, **caractérisé en ce que** les informations spatiales sont obtenues en détectant l'unité photo-détectable des bases nucléiques.

4. Procédé selon l'une quelconque des revendications 1 à 3, **caractérisé en ce que** les informations spatiales sont obtenues par séquençage par synthèse.

5. Procédé selon l'une quelconque des revendications 1 à 4, **caractérisé en ce que** l'échantillon de tissu est enlevé de la surface.

6. Procédé selon l'une quelconque des revendications 1 à 5, **caractérisé en ce que** le brin d'ADNc est circularisé puis multiplié par une polymérase capable d'amplification de cercle roulant en une pluralité de concatémères d'ADN.

7. Procédé selon l'une quelconque des revendications 1 à 6, **caractérisé en ce que** les molécules d'ancre sont distribuées au hasard sur le substrat solide avec une densité correspondant à la concentration de la molécule.

8. Procédé selon l'une quelconque des revendications 1 à 6, **caractérisé en ce que** l'échantillon de tissu est perméabilisé après l'avoir fourni à la surface.

9. Procédé selon l'une quelconque des revendications 1 à 8, **caractérisé en ce que** la molécule d'ancre comprend au moins une séquence d'amorce pour la polymérase capable d'amplification de cercle roulant.
